# EUROPEAN PATENT APPLICATION

(11) **EP 2 949 664 A1**
(43) Date of publication of application: **02.12.2015**
(21) Application number: 14743392.4
(22) Date of filing: 22.01.2014
(51) Int. Cl.: C07K 14/47, C12N 15/12, C12N 15/01, C12N 15/63, A61P 9/00, A61P 9/04, A61P 9/06, A61P 9/10, A61P 3/00

(54) **MG53 MUTANT AND MUTATION METHOD AND USE THEREOF**

(30) Priority: 25.01.2013 CN 201310030960
(71) Applicant: Beijing Boyalife Pharmaceuticals Ltd., Beijing 100085 (CN)
(72) Inventor: XIAO, Ruiping, Beijing 100871 (CN); CAO, Chunmei, Beijing 100871 (CN); ZHANG, Yan, Beijing 100871 (CN); LV, Fengxiang, Beijing 100871 (CN)
(74) Representative: Gill, Siân Victoria
(86) International application number: PCT/CN2014/000078
(87) International publication number: WO 2014/114184

(57) **Abstract**

Disclosed in the present invention is a MG53 mutant, wherein any one or two or more of 7 cysteines in the RING domain at the N-terminal of MG53 are mutated into mutations of non-polar amino acids. The MG53 mutant has preventive / therapeutic effects and uses in the protection of the heart, and the treatment of heart diseases caused by cell death, while avoiding the side effects caused by MG53 such as insulin resistance, obesity, and diabetes, etc.

## Description

### Technical Field

The present invention relates to a MG53 mutant, a use of pharmaceutical composition comprising the MG53 mutant in protecting the hearts and preventing/treating cardiac diseases induced by cell death. Particularly, although both wild type and mutated MG53 can protect the hearts, unlike wild type MG53, MG53 mutant avoids the side-effects such as resistance, obesity and diabetes.

### Background Art

MG53 (mitsugumin 53, often called as MG53 in abbreviation or TRIM72) is a muscle specific protein of tripartite motif family (TRIM) consisting of three unique motifs as to the RING, the B-BOX, and the coiled-coil domain. The three domains combine together and bind to unnecessary proteins to afterward induce its degradation through ubiquitination. MG53 is also an essential factor of cell membrane repair mechanism.

In medical therapy aspect, the MG53 treatment for cardiac diseases induced by cell apoptosis has been well-recognized and accepted by experts of the field. However, the increase of MG53 poses an inevitable danger of bringing out insulin resistance, obesity and metabolic disorders in cardioprotection. That means MG53 will provoke insulin resistance, obesity and metabolic disorders along with cardioprotection. This is a dilemma which scientists are concerned and has not been solved yet.

### Summary of the Invention

The present invention relates to a MG53 mutant, in the N-terminus of which any one or two or more than two of the seven cysteine residuals in the RING domain are substituted by non-polar amino acids. The MG53 mutant is capable of preventing and treating cardiac disease induced by cell death. Particularly, unlike the wild type MG53, the MG53 mutant also avoids the side effects such as leading to insulin resistance, obesity and diabetes.

The MG53 can protect hearts from cardiac diseases by elevating MG53 level, but the increase of MG53 also causes insulin resistance, obesity and metabolic disorders meanwhile. To get rid of these side effects, the inventor has carried out a great amount of scientific work. The main objective is to mutate MG53, in the hope of constructing a MG53 mutant which has the function of cardioprotection without the side effects.

The present invention discloses a MG53 mutant, wherein any one or two or more than two of the seven cysteine sites of the RING domain of the N-terminus are substituted by non-polar amino acids. The seven cysteines situate at the 14^{th}, 17^{th}, 29^{th}, 34^{th}, 37^{th}, 53th, 56^{th} sites of the RING domain.

The seven cysteine residuals are the key sites of the RING domain, also the RING domain is the essential compartment of the MG53 to act as E3 ligase in degrading the subtract and induce insulin resistance, obesity , diabetes and metabolic syndrome. So the inventor chooses these seven cysteines as his research subject.

All the experiments and facts indicate that if any one or two or more than two of the seven cysteine sites are replaced by non-polar amino acids, it will come up with the desired outcome and the beneficial effects.

The present invention presents a MG53 mutant, which avoids MG53-mediated insulin resistance, obesity, diabetes, metabolic syndrome while its cardio-protective function is kept.

The non-polar amino acids within the MG53 mutant as indicated above maybe alanine, glycine, valine, leucine, isoleucine, proline, phenylalanine, tryptophan or methionine. More preferably it may be alanine, glycine, leucine, proline, valine or isoleucine. Most preferably, the non-polar amino acid may be alanine.

When the non-polar amino acid of MG53 mutant is alanine, the MG53 mutant of the claim may be any of MG53C14A, MG53C17A, MG53C29A, MG53C34A, MG53C37A, MG53C53A and MG53C56A.

The gene sequence of the MG53 mutant indicated in the present invention may come from primates, rats or mice.

In this experiment, the inventor selects mRNA of the wild-type MG53 (TRIM72) (NCBI: NM_001079932.3) from mice, alternatively it may come from:
Human: mRNA: NM_001008274.3, CDS: NP_001008275.2;
Rats: mRNA: NM_001077675.1, CDS: NP_001071143.1;
Monkeys: mRNA: XM_001112866.2, CDS: XP_001112866.1;

It is not limited to species as mentioned above, all MG53 expressing species shall be taken into account, and correspondent MG53 mutant proteins are based on the sequences as mentioned above.

The most preferential option indicated in the present invention is the MG53 mutant (MG53 mutant protein) with the 14^{th} cysteine of the RING domain in the N-terminus substituted by alanine. As the substituted cysteine is the 14^{th} cysteine, the MG53 mutant is MG53C14A.

The inventors are quite surprised that the 14^{th} cysteine site of MG53 is essential for MG53-meditated insulin resistance, obesity and diabetes as further research progresses, which means that the MG53 mutant (MG53C14A) cannot give rise to insulin resistance, obesity and metabolic syndrome. However, MG53C14A still can protect hearts from myocardial injury. (Reliable data support the conclusion). That means MG53C14A could protect hearts in the premise of not bringing about insulin resistance, obesity and diabetes. It is a desirable but unexpected result that the inventor is looking for. If necessary, the supplemented data of the pharmacological tests of the present invention would be submitted later to support the contention.

The present invention chooses an alanine substitution as a preferential example to formulate the MG53 mutant hereinafter. To be specific, alternative replacement of cysteine is not just limited to A(alanine), eight other non-polar amino acids could be candidates, such as glycine, leucine, proline, valine or isoleucine, et al. All those mutants could exert the function of cardioprotection without bringing out insulin resistance, obesity, diabetes and metabolic syndrome. Due to constraints of space, here the MG53C14Awas selected as the most preferential example. All mutation methods of alternative mutants are similar to MG53C14A.

MG53 is a newly found member of the super family of tripartite motif-containing proteins which only expresses in skeletal muscle and myocardium. Early studies demonstrate that MG53 in skeletal muscle and myocardium aids to repair cell membrane injury as well as regulates the transportation of cellular vesicles and the regeneration of skeletal muscle as structural proteins. Besides, MG53 is able to mediate the association of Caveolin-3 and PI3K to activate the reperfusion injury salvage kinase pathway (RISK pathway), thus MG53 plays an important role in protecting hearts during heart ischemia preconditioning. The western blots have showed that significant upregulation of skeletal muscle specific MG53 appears in multiple insulin resistance animal models, which suggests MG53's possible role in inducing insulin resistance.

Amino acid sequence analysis shows that MG53 consists of the RING domain (RING finger domain), B-box domain/ Coiled-coil domain and SPRY domain. And only the RING domain shows the activity of E3 ubiquitin ligase. So the inventor deems the E3 ubiquitin ligase activity is RING domain-dependent. The first cysteine which binds to the Zn²⁺ of Zn²⁺ finger structure in the RING domain (the 14^{th} cysteine site of the MG53 peptide from wild type mice) is key factor for E3 ubiquitin ligase activity of RING domain. The inventor constructs two MG53 mutants which the E3 ubiquitin ligase is blocked, one is the RING domain deleted (ΔRING-MG53), the other one is the 14^{th} cysteine mutated to an alanine (C14A-MG53). Compare these two mutants to the wild type MG53 in terms of the impact on IRS1 ubiquitination, the inventor finds that only wild type MG53 can effectively ubiquitinate insulin receptor(IR) and insulin receptor subtract1 (IRS1) while ΔRING and C14A cannot. This strongly proves the essential role of the MG53 RING domain, especially the 14^{th} cysteine site in catalyzing the ubiquitination of IR and IRS1. Similar to MG53 C14A, ΔRING-MG53 is also able to protect hearts from the myocardial injury induced by hypoxia. The function of ΔRING-MG53 in cardioprotection is first observed by the public.

This discovery promotes inventor to deepen the investigation of the underlying mechanism of MG53 in insulin resistance of skeletal muscle. These findings and conclusions mean it is not a conjecture or assumption of science, but real research accomplishments in the industrialization and a true invention.

To further discuss the role of MG53 in degrading IR and IRS1 and down regulating insulin signaling pathways of skeletal muscle, as well as to figure out the underlying mechanism of MG53 mediated ubiquitination and degradation of IR and IRS1, the inventor carried out in-depth researches. Overexpression of wild type MG53 and its two mutants in C2C12 cells for the impact on insulin signaling shows that only wild type MG53, which could degrade the IR and IRS1, is capable of blocking the insulin signaling pathways, while the two mutants are E3 ubiquitin ligase blocked, especially for C14A with only one amino acid mutated, cannot block the insulin signaling pathways.

That is to say, the early conclusions of the present invention are: Firstly, the abnormal overexpression of MG53 causes the onsets of insulin resistance, obesity, diabetes and metabolic syndrome; Secondly, it is the first time to prove that MG53 constitutes the E3 ubiquitin ligase for IR and IRS1 which activate the ubquitin-proteasome pathway to degrade IR and IRS1 in skeletal muscle and is required for insulin resistance, further leads to obesity, diabetes and metabolic syndrome. Thirdly, the inventor can make further associated thinking.

E3 ligase is the only controlled factor in protein ubiquitination. E3 ligase can be divided into two categories based on the binding style of the ubiquitin with target proteins: RING (really interesting new gene) domain E3 ligase and HECT (homologous to E6-associated protein C terminus) domain E3 ligase, the former E3 ligase simultaneously binds ubiquitin-carried E2 and the target proteins, The latter E3 ligase transfers the ubiqutin from E2 to a cysteine site of HECT domain, followed by transferring the thioeaster-ubiquitin to the target proteins. The ubiquitin itself consists of 76 amino acids with scattered seven lysine residuals, where the seven cysteine residuals bind correspondently, which will covalently bind to other ubiquitin, sequentially prolongs the ubiquitin chain after several cycles of bindings, this will enhance the .recognition of the labeling. It is generally accepted that a chain of 48 lysine-meditated ubiqutin is a classic guiding signal that initiates the target protein degradation; however, the chain of 48 lysine-meditated ubiqutin will meditate the non-degradation pathways of the target protein. However, this theory seems to be revised. The ubiqutin-target protein locates itself in the 26S proteasome with ubiquitin-binding domains (UBDs), followed by degradation through ubiquitination and unfolding. As science advances, people are getting more information about the overall structure of the insulin signaling network in the insulin targeted organs, 'the IR/IRSs-PI3K-Akt pathways'. However, it is just an initial step to explore the underlying mechanism of the insulin signaling. The inventor found during research that, in addition to the ubiquitous phosphorylation regulation, the protein degradation also plays a remarkable role in the insulin signaling. Early in the insulin resistance studies, it has been observed that the tyrosine phosphorylation of IR and IRS1 is universally suppressed and the levels of the relevant proteins decline in patients and animal models. Recent years scientists are getting more insights into the IRSs degradation mechanism, for instance, SOCS1/3 acts as a subtract recognizer of the culling-RING type ubiquitin ligase to mediate the degradation of IRS1 and IRS2. It is also found that SOCSs are notably upregulated upon the stimulation of multiple inflammatory factors. Combined with lots of clinical data and animal evidences, scientists realize that obesity and diabetes 2 is substantively a state of inflammation, where numerous factors actively participate in the insulin resistance. In addition, it is also proved that Akt and its downstream factors possibly become the targets of UPS in tissues and are degraded, which subsequently influences the glucose transport and gluconeogenesis. However, more studies are still needed to explore how IR degrades during insulin resistance.

In brief, this invention provides a MG53 mutant protein supported by massive data that one or two or more than two of the seven cysteine residuals in the N-terminus of the RING of the MG53 mutated to alanines comprising MG53C14A, MG53C17A, MG53C29A, MG53C34A, MG53C37A, MG53C53A, MG53C56A and others not mentioned , the most preferential option is MG53C14A.

The present invention provides a method of constructing the mutation of MG53, which adopts a point-mutation kit to mutate the sequence of wild-type MG53 plasmid, to obtain the MG53 mutant plasmid. The kit is Easy Mutagenesis System marketed by Beijing TransGen Biotech.

An Easy Mutagenesis System point-mutation kit (Beijing TransGen Biotech, China) was used to mutate the 14^{th} cysteine of the wild type MG53 into a non-polar amino acid. When the amino acid is alanine, which means the 14^{th} cysteine is mutated into alanine, it is called MG53C14A.

By the same method, the inventor also constructs other MG53 mutants with mutations from the cysteine to glycine, leucine, proline, valine and isoleucine so as to obtain the MG53C14G, MG53C14L, MG53C14P, MG53C14V, and MG53C14I.

In the embodiments, the mutation protocol is:
1. Design the upperstream and downstream primers, which contains the mutation site, and the length of overlapping region is 18-27bp;
2. Amplify the MG53 gene sequence by PCR reaction with DNA polymerase with wild-type MG53 plasmid or cDNA of MG53 as the template. The PCR product is confirmed by agrose gel electrophoresis. The to-be-mutated plasmid is wild-type MG53 plasmid.
3. The PCR product is digested by restrictive enzyme DpnI.
4. Transform the restrictive cut product into E.coli competent TOP10: thaw the competent TOP10, followed by adding PCR product into the cell and incubate on ice, followed by adding LB medium and shaking culture, then spray the cells on LB medium plate with antibiotics, select the single colony for DNA sequencing, the positive colony means successful construction of the MG53 mutant plasmid.
5. The MG53 mutant protein will be obtained by transfecting the mutated MG53 plasmid into cells by ScreenFectA or Lipofectamine.

ScreenFectA (Incella) is a kit for commercial use, which provides the reagents needed and the detailed protocol.

The kit of Incella consists of ScreenFect®A transfection reagent and ScreenFect®A Dilution Buffer.

The preferential overlapping region in step 1 is 20bp.

In another preferential example, QuikChange II point mutation kit is used to mutate the 14^{th} cysteine of the Flag-MG53 plasmid to alanine to obtain the Flag-C14A MG53.

In another preferential example , the method of constructing the mutation is as follows:
1. Design the upperstream and downstream primers, which contains the mutation site, and the length of overlapping region is 18-27bp;
2. Amplify the MG53 gene sequence by PCR reaction with DNA polymerase with 200 ng wild-type MG53 plasmid or cDNA of MG53 as the template. The PCR product is confirmed by agrose gel electrophoresis.
   The PCR program is as follows: 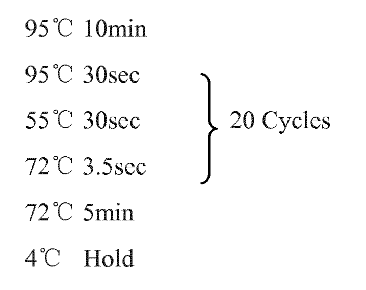
3. The PCR product is digested by restrictive enzyme DpnI, where the reaction is as follows: add 1ul DpnI to 10ul PCR product at 37 °C for overnight digestion.
4. Transform the restrictive cut product into E.coli competent TOP10: thaw the competent TOP10, followed by adding PCR product into the cell and incubate on ice for 30min, then heatshock the mixture at 42°C for 60s , followed by adding LB medium and shaking culture for 45min, then spray the cells on LB medium plate with antibiotics and incubate at 37°C overnight.
5. Select the single colony for DNA sequencing, the positive colony means successful construction of the MG53 mutant plasmid.

### Reference documents:

*Strata Gene'QuikChange Site-directed Mutagenesis kit. TransGen Biotech Easy Mutagenesis System. Detailed Protocol:*
*http:*//*www.transgen.com.cn*/*uploadfile*/*201111*/*20111109161357731.pdf.*

For more information about the method of constructing the mutation of the MG53 mutants, please refer to the protocol provided by Beijing TransGen Biotech in its Easy Mutagenesis System kit.

The present invention provides a use of pharmaceutical composition comprising MG53 mutants in treating myocardial injury.

The present invention provides a use of the pharmaceutical composition in treating myocardial injury disease including insulin resistance induced by myocardial injury, further treating the diseases including myocardial ischemia injury, myocardial ischemia/reperfusion injury, myocardial infarction, heart failure, cardiac arrhythmia and cardiac rupture.

The present invention particularly provides a MG53 mutant, MG53C14A, as well as a use of pharmaceutical composition comprising MG53C14A in treating myocardial injury.

The use of the MG53 in treating myocardial injury, preventing/treating myocardial ischemia and reperfusion injury has been disclosed by the patent CN 200910241451.3.

Overexpression of the MG53 and MG53C14A in myotubes both has the effect of protecting the cells from the hypoxia induced injury.

The term 'MG 53 mutant' referred in this application means the mutated MG53 protein or MG53 mutant protein.

Insulin resistance is a fundamental pathogenic factor present in various metabolic disorders including obesity and type 2 diabetes. Although skeletal muscle accounts for 70-90% of insulin-stimulated glucose disposal, the mechanism underlying muscle insulin resistance is poorly understood. Here the inventor shows in mice that muscle-specific mitsugumin 53 (MG53; also called TRIM72) mediates the degradation of the insulin receptor (IR) and insulin receptor substrate 1 (IRS1), and when upregulated, causes metabolic syndromes featuring insulin resistance, obesity, hypertension and dyslipidaemia. MG53 expression is markedly elevated in models of the insulin resistance, and MG53 overexpression suffices to trigger muscle insulin resistance and metabolic syndrome sequentially. Conversely, ablation of MG53 successfully prevents diet-induced metabolic syndrome by preserving the insulin receptor, IRS1 and insulin signaling integrity. Mechanistically, MG53 acts as an E3 ligase targeting the insulin receptor and IRS1 for ubiquitin-dependent degradation, comprising a central mechanism controlling insulin signal strength in skeletal muscle. These findings define MG53 as a novel therapeutic target for treating metabolic disorders and associated cardiovascular complications.

A cluster of disorders known as metabolic syndrome, including insulin resistance, central obesity, dyslipidaemia and hypertension, is increasing at epidemic rate and has become one of the most serious threats to human health. Metabolic syndrome increases the risk of developing cardiovascular disease two-fold, and the risk of type 2 diabetes five-fold. Insulin resistance is a fundamental pathogenic factor shared by a myriad of metabolic disorders including metabolic syndrome, obesity and type 2 diabetes. Because skeletal muscle is responsible for 70-90% of insulin-stimulated glucose disposal, insulin resistance in skeletal muscle probably has a central role in the pathogenesis of metabolic syndrome and resultant type 2 diabetes. Indeed, longitudinal studies have provided evidence that skeletal muscle insulin resistance is the earliest step in the pathogenesis of the metabolic syndrome and type 2 diabetes. However, the mechanism underlying skeletal muscle insulin resistance is poorly understood.

Representative western blots and averaged data show the upregulation of MG53 in skeletal muscle from rodent and non human primates (NHP) models of insulin resistance and metabolic disorders, versus skeletal muscle from their respective age- and gender-matched controls. Data are normalized to GAPDH. The survey reveals that abundance universally increases in high-fat diet (HFD)-induced obese mice, db/db diabetic mice, spontaneously hypertensive rats, and non human primates with metabolic syndrome. The upregulation of MG53 was also confirmed in obese humans (supplementary). These results provided the critical clue for a previously unappreciated link between MG53 and metabolic diseases.

To determine whether MG53 is required for the pathogenesis of the metabolic syndrome, the inventor used MG53-deficient (MG53^{-/-}) mice and their wild-type littermates to track from 3 weeks of age, changes in their body weight and metabolic parameters in response to a HFD (60% calories from fat) or chow. Compared to wild-type mice, MG53^{-/-}mice on chow showed no phenotypic difference in body weight, blood pressure, serum cholesterol and triglyceride levels from 3 to 38 weeks of age, except for a significant reduction in blood glucose concentration without changing the serum insulin level (measured at 38 weeks).

Using dietary intervention, however, inventor found profound differences between MG53^{-/-}and wild-type mice. After 35 weeks on the HFD, with upregulated MG53, wild-type mice developed metabolic syndrome featuring obesity and hypertension, hyperglycemia, hyperinsulinaemia, dyslipidaemia and hepatosteatosis. Notably, MG53^{-/-} mice are resistant to the HFD-induced metabolic disorder. After the HFD for 35 weeks, blood pressure, blood glucose and serum insulin and lipid (cholesterol and triglyceride) levels in MG53^{-/-} mice are largely comparable to those of wild-type or MG533^{-/-} mice on chow. MG53 deficiency also markedly attenuated HFD-induced obesity, hepatosteatosis, lipid accumulation in skeletal muscle, adipocyte hypertrophy, and the increases in both white and brown fat weight.

To track whole-body insulin sensitivity in relation to the development of metabolic syndrome, the inventor performed glucose tolerance tests (GTTs) and insulin tolerance tests (ITTs) at various time points after dietary intervention. The HFD led to glucose intolerance and insulin resistance in wild-type mice at as early as 7 weeks, that is, before the onset of obesity at 11 weeks. As the HFD treatment was continued, both glucose intolerance and insulin resistance are progressively exacerbated in wild type mice. After 35 weeks, the HFD caused compensatory hypertrophy of pancreatic islets a seven fold increase in basal serum insulin concentration, and failure of glucose to stimulate insulin secretion.

Notably, MG53^{-/-}mice on the HFD showed none of these phenotypes, maintaining normal blood glucose and insulin levels even after 30 weeks of the HFD. Changes in pancreatic morphology and the insulin secretion response are also markedly ameliorated in MG53^{-/-}mice. Thus, the MG53 ablation protects mice against HFD-induced insulin resistance and the sequel of metabolic disorders, indicating that MG53 is required for HFD-induced insulin resistance and metabolic syndrome.

To determine further whether the upregulation of MG53 is sufficient to initiate the pathogenesis of metabolic disorders, the inventor generated transgenic mice over expressing MG53 (MG53^{Tg}). MG53 protein levels are increased by 2.6+0.3-fold in skeletal muscle and 2.6+0.7-fold in the heart at 38 weeks of age. However, there was no detectable expression of MG53 in non-muscle tissues (lung, brain, hypothalamus, liver, intestine, kidney, visceral fat and testis) in MG53^{Tg} mice, attesting that MG53 expression is tightly regulated in a muscle-specific manner. Even in the absence of dietary intervention, MG53^{Tg} mice at 38 weeks of age are obese and hypertensive, along with dyslipidaemia, hyperinsulinemia and increased fasted blood glucose levels. Energy expenditure during light and dark was significantly lower, but daily food intake was unchanged in MG53Tg mice relative to wild-type littermates. Notably, GTTs and ITTs revealed severe impairments in glucose metabolism and insulin sensitivity in MG53^{Tg} mice, which are accompanied by pancreatic islet hypertrophy and failure of glucose-stimulated insulin secretion. Anatomical and histological data further documented that MG53Tg mice had central obesity, hepatosteatosis, enlargement of adipocytes, and lipid accumulation in skeletal muscle. These data indicate that the upregulation of MG53 is both necessary and sufficient to trigger insulin resistance that further develops into metabolic syndrome. Annotations: GTTs and ITTs are performed in wild-type and MG53^{-/-} mice on chow or the HFD at indicated time points. Haematoxylin and eosin staining of the pancreas. Glucose (2gkg⁻¹, intraperitoneally) stimulated changes in serum insulin concentrations. Wild-type and MG53^{-/-} mice are on chow or the HFD for 35 weeks. Data are mean±s.e.m.

Upon insulin stimulation, the intrinsic tyrosine kinase of the insulin receptor leads to receptor auto phosphorylation at tyrosine residues. Subsequent recruitment and phosphorylation of the insulin receptor substrates such as IRS1 and IRS2 is the pivotal event which, in turn, activates the downstream phosphatidylinositol-3-OHkinase (PI (3) K)-Akt-GSK3β signaling pathway to regulate glucose homeostasis in skeletal muscle. To decipher the molecular mechanism underlying theMG53-mediated insulin resistance, the inventor investigated possible MG53-mediated regulation of the insulin receptor-IRS1-PI (3) K-Akt-GSK3β signaling cascade. In the MG53^{Tg} model of insulin resistance and metabolic disorders, insulin-stimulated tyrosine phosphorylation of the insulin receptor (β subunit) and IRS1 and Ser 473 phosphorylation of the Akt in skeletal muscle are abrogated. The protein levels of the insulin receptor and IRS1 are markedly reduced, whereas their messenger RNA levels remained intact in skeletal muscle from these mice. To distinguish the direct effects of the MG53 overexpression from adaptive responses, the inventor performed adenoviral gene transfer of MG53 in cultured C2C12 myotubes. A 3.5±0.2-fold increase in MG53 over baseline levels led to marked reductions in the insulin receptor and IRS1 protein levels. But not their mRNA levels. MG53 overexpression also blocked the insulin-induced activation of the insulin receptor, IRS1, Akt and GSK3-β indicating that the upregulation of the MG53 at the cellular level recaptures the salient features of MG53^{Tg} mice. Thus, our in vivo and in vitro data indicate that elevated MG53 expression is sufficient to cause simultaneous downregulation of the insulin receptor and IRS1, thus suppressing the insulin signal transduction at two crucial steps. Annotation: Overexpression of MG53 triggers systemic insulin resistance and Metabolic syndrome, Body weight of wild-type and MG53^{Tg} mice. Systolic and diastolic blood pressure, serum cholesterol, triglyceride, serum insulin and blood glucose levels in the fasted and fed state of wild-type and MG53^{Tg} mice at the age of 38 weeks. GTTs and ITTs in wild type and MG53^{Tg} mice at the age of 30 weeks, Insulin concentrations or their relative changes as a percentage of respective baselines in response to glucose (2 g kg⁻¹, intraperitoneally) in wild-type andMG53^{Tg} mice at the age of 38 weeks.

Similar to MG53 overexpression, the HFD, which elevated MG53 expression level, profoundly suppressed skeletal muscle insulin signaling in wild-type mice and simultaneously reduced insulin receptor and IRS1 protein levels without altering their mRNA levels. Moreover, post-transcriptional downregulation of both the insulin receptor and IRS1, accompanied by MG53 upregulation, is a common feature shared by all of the rodent models of insulin resistance used in this study, as is the case in animal models and humans with obesity or type 2 diabetes. In more stringent tests, the inventor showed thatMG53 ablation enabled mice to maintain insulin receptor and IRS1 integrity as well as whole-body insulin sensitivity, even under the metabolic stress induced by the HFD. Specifically, the HFD failed to decrease the insulin receptor and the IRS1 abundance in MG53^{-/-}mice. In fact, genetic ablation of MG53 led to an evident accumulation of the insulin receptor and IRS1 in skeletal muscle, perhaps contributing to the decrease in blood glucose level in MG53^{-/-} mice relative to wild-type littermates. Concomitantly, the insulin-induced phosphorylation of the insulin receptor, IRS1, Akt and GSK3-β was significantly greater inMG53^{-/-}than that in wild-type mice on chow. Hence, MG53 upregulation seems to be indispensible for the HFD- and metabolic-disease-associated downregulation of the insulin receptor and IRS1 in skeletal muscle.

Next, the inventors sought to determine how MG53-mediated, muscle-specific insulin resistance develops into whole-body metabolic disorders. For this purpose, the inventors tracked the onset of various facets of metabolic disorders in multiple organs in MG53^{Tg} mice and HFD-treated wild-type mice. The present results showed that skeletal muscle insulin resistance induced by overexpression of MG53 and the HFD preceded the development of whole-body metabolic disorders including obesity and multi-organ insulin resistance, not the other way round. In MG53^{Tg} mice at 6 weeks of age, with body weight comparable to wild-type controls. The insulin signaling was clearly impaired in skeletal muscle, but not liver and visceral fat tissues. Likewise, the HFD in wild-type mice led to significant MG53 upregulation and a muscle-specific insulin signaling defect at as early as 1 week of dietary intervention, before the manifestation of systemic glucose intolerance and insulin intolerance at 7 weeks and appreciable obesity at 11 weeks on the HFD. Later, liver and fat tissues developed full-fledged insulin resistance in both the MG53^{Tg} mice on chow (at 38 weeks of age) and wild-type mice on the HFD (35 weeks of dietary intervention). Together with the profound protective effects of MG53 ablation on metabolic disorders, these data strongly suggest that MG53-mediated muscle insulin resistance is a major factor in the instigation of the pathogenic process. In line with the present findings, previous studies on humans and animal models have also suggested an essential role of reduced muscle insulin signaling in the pathogenesis of obesity and glucose intolerance, albeit not without controversy. Annotation: Representative western blots and averaged data showing insulin-induced tyrosine phosphorylation of insulin receptor-β subunit (IR-β) and IRS1, serine phosphorylation of the Akt and their total protein levels in skeletal muscle from Wild-type and MG53^{Tg} mice at the age of 38 weeks, or MG53^{-/-}mice and their wild-type littermates on chow or the HFD for 35 weeks. The phosphorylated and total proteins are normalized to GAPDH and presented as fold of their respective wild-type baselines, Typical western blots and statistical data showing insulin-induced phosphorylation of the Akt in skeletal muscle (SM), liver and visceral fat (Fat) from wild-type and MG53Tg mice at the age of 6 weeks or 38 weeks.

Because IRS1 is a nodal point shared by insulin receptor- and insulin like growth factor-1 (IGF-I)-receptor-mediated signaling pathways, the inventor investigated the effects of the MG53 on muscle IGF-I signaling in addition to insulin signaling. By comparing the dose-response of insulin- or IGF-I-induced tyrosine phosphorylation of IRS1 in skeletal muscle from wild-type andMG53-deficient mice, the inventor found that MG53 preferentially targets IRS1 in the setting of insulin signaling. Specifically, whereas MG53 ablation augmented the whole dose-response of tyrosine phosphorylation of IRS1 by insulin stimulation, it potentiated the effect of IGF-I only at high but not low IGF-I concentrations. If MG53 selectively targets insulin-receptor-mediated IRS1 signaling, the apparent regulation of IGF-I receptor signaling by MG53 might be attribute to heterodimerization of the insulin receptor and IGF-I receptor or cross-activation of insulin receptor by high dosage of IGF-I.

Because MG53 contains a canonical E3 ligase RING finger domain at the amino terminus, the inventor proposed that it may function as a muscle specificE3 ligase targeting the insulin receptor and IRS1 for ubiquitin-dependent degradation. Multiple lines of evidence support this hypothesis. First, co-immunoprecipitation revealed a physical interaction of endogenous MG53 with the insulin receptor and IRS1 1 in skeletal muscle in the presence or absence of insulin stimulation, and of ectopically expressed MG53 with the insulin receptor and IRS1 in HEK 293 cells (Supplementary. Second, over expression of MG53 abundantly increased the ubiquitination of both the insulin receptor and IRS1 in skeletal muscle from MG53Tg mice. Furthermore, the HFD profoundly augmented the insulin receptor and IRS1 ubiquitination in skeletal muscle in vivo in wild-type, but not MG53^{-/-}mice. The present findings demonstrate thatMG53 E3 ligase facilitates the ubiquitin-dependent degradation of both the insulin receptor and IRS1 in skeletal muscle, although several other E3ligases have been previously implicated in IRS1 turnover in certain cell culture systems and tissues with FBXO40 being muscle specific. In contrast, the protein abundance of skeletal muscle IRS2, GLUT1 orGLUT4, which are also involved in glucose homeostasis, was unaffected by either MG53 over expression or its ablation, indicating that they are not the substrates for MG53.

Sequence analysis predicted that the cysteine-rich RING finger (especially seven cysteines) domain in the N-terminus of MG53 and, in particular, the first cysteine residue (cysteine 14) which binds Zn²⁺ may be crucial for MG53 E3 ligase activity in light of previous investigations into other RING-type E3 ligases. Indeed, the RING finger deletion (DRING) or the alanine substitution of the cysteine 14 (C14A) abolished or markedly attenuated the effects of MG53 on insulin receptor and IRS1 ubiquitination. Notably, these MG53 mutants affected neither the protein levels of the insulin receptor and IRS1 nor the insulin-induced activation of the insulin receptor-IRS1-Akt-GSK3β signaling cascade. Thus, at the molecular level, the RING finger domain is required for MG53 E3 ligase activity. Furthermore, proteasome inhibition by clasto-lactacystin-β-lactone(β-lac) abolished MG53-induced downregulation of the insulin receptor and IRS1, restored tyrosine phosphorylation of the insulin receptor and IRS1 and Ser 473 phosphorylation of Akt as well as insulin-induced glucose uptake, indicating the involvement of the proteasome system in MG53-mediated suppression of insulin signaling. Similar results are obtained with another proteasome inhibitor, MG132. Taken together, in vivo and in vitro data demonstrate that MG53 acts as a novel E3 ligase that directly regulates the insulin receptor and IRS1 protein stability through ubiquitin-dependent degradation. This finding identifies MG53 as a mechanism underlying the simultaneous downregulation of the insulin receptor and IRS1 in the context of systemic insulin resistance and metabolic diseases.

In summary, whereas MG53 is known for its roles in membrane repair and cardio protection, the inventor have now shown that MG53 is universally upregulated in animal models with insulin resistance and metabolic disorders, and that muscle-specific MG53 upregulation is necessary and sufficient to trigger whole-body insulin resistance and syndrome. Mechanistically, MG53 acts as a novel muscle specific E3 ligase targeting both the insulin receptor and IRS1 for the ubiquitin-dependent degradation, hence constituting a crucial negative regulator of insulin signaling in skeletal muscle which, in turn, triggers systemic defects in insulin signaling and metabolism.

These findings not only define MG53 as a negative regulator of skeletal muscle insulin sensitivity, but also establish MG53-mediated suppression of muscle insulin signaling as a central mechanism underlying whole-body insulin resistance and metabolic syndrome, marking MG53 E3 ligase as a potentially important therapeutic target for the treatment of diverse metabolic diseases, including obesity, type 2 diabetes and associated cardiovascular complications.

### Methods Summary

Reagents and materials: Antibodies to PY100, p-Akt, Akt, p-GSK3β and IRS1 are from Cell Signaling Technology; anti-MG53 and GLUT1 antibodies are from Abcam; p-IR-β and IRS1 antibodies are from Upstate; IR-β, GAPDH, GSK3-3β and GLUT4 antibodies are from Santa Cruz Biotechnology. MG132, clasto-lactacystin β-lactone (β-lac), anti-b-actin, Flag, Myc and insulin antibodies are from Sigma-Aldrich. 2-(N-(7-nitrobenz-2-oxa-1, 3-diazol-4-yl) amino)-2-deoxyglucose (2-NBDG) was from Invitrogen. Human IGF-I (Human insulin-like growth factors-I) was from PeproTech. Unless indicated otherwise, all chemicals are from Sigma-Aldrich.

Animal models with insulin resistance: db/db mice (male db/db mice at 25 weeks of age) and lean control mice are from the Jackson Laboratory. Spontaneously hypertensive rats (male SHRs at 12 months of age) and Wistar Kyoto rats (WKY) are from Vital River Laboratories, Beijing, China. The development and characterization of a nonhuman primate (NHP) model of spontaneous insulin resistance and metabolic syndrome was reported previously.

MG53^{-/-} mice and dietary intervention: All animal procedures and euthanasia are performed in accordance with protocols approved by the Committee for Animal Research of Peking University, China, and conformed to the Guide for the Care and Use of Laboratory Animals (NIH publication No. 86-23, revised 1985).All mice are maintained in a temperature-controlled barrier facility with a 12-h light/dark cycle and are given free access to food and water in the Center for Experimental Animals at Peking University, Beijing, China (an AAALAC-accredited experimental animal facility). Only male animals are used in this study. The generation of MG53^{-/-} mice was described previously. Dietary intervention with a high-fat diet (60% calories from fat, Cat. #D12492, Research Diets Inc.) Or a chow diet (11.4% calories from fat, Academy of Military Medical Sciences, China) started from 3 weeks of age in MG53^{-/-} mice and wild-type littermates.

MG53^{Tg} mice: The full-length murine MG53 cDNA coding sequence was cloned into the XhoI site of pUC-CAGGS, under the regulation of the chicken-actin promoter. After linearization with SalI and subsequent gel-purification, this construct was microinjected into the pronuclei of fertilized C57BL/6J mouse eggs. PCR was used for genotyping.

Plasmids and adenoviral vectors: DNA fragments corresponding to full-length or RING (deletion of the ΔRING domain) of MG53 are amplified from a mouse cDNA library by PCR and inserted into p3 X FLAG-CMV-10 Expression Vector (Sigma-Aldrich) using the BgIII and XbaI restriction sites. Full-length MG53 sequence was also inserted into pcDNA4/TO/Myc-His B expression vector (Invitrogen) using the KpnI and XhoI restriction sites. The C14A MG53 mutant (the 14th cysteine substituted by alanine) was generated from the wild-type MG53 construct (FL-MG53) by point mutation using Stratagene's QuikChange II site directed mutagenesis kit. The insulin receptor sequence (IRS) was subcloned from pBABE-bleo human insulin receptor B (Addgene) and inserted into pcDNA4/TO/Myc-His B expression vector (Invitrogen) using the HindIII and XbaI restriction sites. IRS1 was subcloned from pBS mouse IRS1 (Addgene) and inserted into pcDNA4/TO/myc-His B expression vector (Invitrogen) using the HindIII and NotI restriction sites. The constructs expressing N-terminal HA-tagged ubiquitin and C-terminal Flag-tagged insulin receptor are provided by D. Chen and I. Leibiger, respectively. Adenovirus expressing GFP or GFP-MG53 was described previously.

Cell culture, adenoviral infection and plasmid transfection; C2C12 myoblasts (from Cell Resource Center, IBMS, CAMS/PUMC) are cultured at 37°C under 5% CO₂ in Dulbecco's modified Eagle's medium (DMEM) supplemented with 10% fetal bovine serum (FBS, Sigma-Aldrich), 0.11 g/L sodium pyruvate, and 1% penicillin-streptomycin. When C2C12 myoblasts reached 90% confluence, gene transfer is performed by adenoviral infection or plasmid transfection. After gene transfer, cells are cultured in DMEM (2% horse serum) for 4 days to differentiate into myotubes.

Cell Hypoxia: cells are cultured in RPMI1640/5 % FBS for 48 hours. Then, the medium was replaced with serum-free RPMI1640 saturated with 95% N₂ /5% CO₂, and cells are placed in a 37 °C airtight box saturated with 95% N₂/5% CO₂ for various periods of time. O₂ concentrations are < 0.1 % (Ohmeda oxygen monitor, type 5120). For normoxia controls, culture medium was changed to RPMI1640 / 5% FCS, and cells are placed in a 37°C / 5% CO2 incubator before analysis.

Determination of Myocardial Injury by:

CellTiter-GloLuminoescent Cell Viability Assay (Cat#G7571, Promega) was used for ATP assay. Details of the method: 1. Mix CellTiter-Glo Substrate (1 tube) and CellTiter-Glo Buffer (1 tube), thawed to room temperature prior to use; 2. Take the cell sample out of the incubator and equilibrate to room temperature prior to use. 3. Adding equal amount of ATP reagent to each wells of cell plate filled with the cell sample; 4. Gentle shake the cell plates for 2 minutes (the sample in this step contains cells, medium and ATP reagents); 5. Equilibrate at room temperature for 10min; 6. transfer the sample into the plate for Luminescent Assay.

LDH was spectrophotometrically assayed with the use of a kit (LDH0360, Shanghai, China), the protocol is as follows: (1).Take out of reagents from the box, mix the reagent 2 with the reagent 1 with a ratio of 1:5 , store at the temperature between 2 and 8°C prior to use; (2). Add each well with 40ul sample; (3). Add each well with 200ul reaction mixture, measure the absorbance of the samples at 340nm.

Co-immunoprecipitation: Tissues or cells are lysed in lysis buffer A (30mM HEPES at pH7.6,100mM NaCl, 0.5% Nonidet P-40, and protease inhibitors mixture) for 30 min at the temperature 4°C, and the lysates are centrifuged at 13,000 r.p.m. for 10 min at 4°C. remove the precipitates, the supernatant of total proteins is ready for use. Wash the protein A beads by ice-cold 1×PBS, then the beads are centrifuged at 4,000 r.p.m for 2min, removed the supernatant, repeat the wash for 3 times , remove the PBS, mix the beads with the protein supernatant and 0.5ug anti-IRS1, the mixture are incubated for 4h at 4°C. Then repeat the step of wash as described previously for 3 times, the beads mix with 1Xloading buffer, and are boiled for 5min at 100°C, then centrifuged at 13,000 r.p.m. for 10 min, the supernatant are loaded on SDS-PAGE and transfer to PVDF. Analysis of tyrosine phosphorylation of IRS1 was performed by immunoprecipitation of IRS 1 with anti-IRS 1 from total lysate, followed by western blot with anti-pTyr antibody (PY100). Other proteins are analyzed with specific antibodies.

Ubiquitination assay: C2C12 myotubes transfected with the indicated plasmids are treated with 10 mM MG132 for 12 hours before collection, then the cells are rinsed in ice-cold PBS, and lysed with RIPA buffer (in mM: 200 NaCl, 20 Tris-Cl at pH8.0, 1 EDTA, 1 EGTA, 1% Nonidet P-40, 0.5% sodium deoxycholate, 0.1% SDS, 2.5 sodium pyrophosphate, 1 β-glycerol phosphate, 1 Na₃VO₄, protease inhibitor mixture and 10 mM MG132) on ice for 10 min, followed by centrifugation at 13,000 r.p.m. for 10 min. The supernatant was pre-cleaned with nProtein A Sepharose 4 Fast Flow (GE Healthcare) for 1 hour, and incubated with anti-Myc antibody and protein A agarose beads for 4 hours at 4°C. The resins are then washed three times with RIPA buffer and resolved onto SDS-PAGE for western blot.

To assay in vivo ubiquitination, skeletal muscles are ground into powders in liquid nitrogen, lysed with RIPA buffer mentioned above for 1 hour at 4°C, and then centrifuged at 13,000 r.p.m. for 10 min. Protein A agarose beads are incubated with indicated antibody for 8 hours at 4 °C, and followed by incubation with a total of 700mg lysate for another 3 hours at 4°C. The immunoprecipitated beads are extensively washed in RIPA buffer, eluted with 2×SDS sample buffer and analyzed by western blots.

Histological analysis: The tissues (liver, pancreas, visceral fat, brown fat and skeletal muscle) for histological analysis are fixed in 4% paraformaldehyde (pH 7.4) overnight, embedded in paraffin, and serially sectioned at 5 um. Standard haematoxylin and eosin staining or immuno fluorescent staining was performed on these sections.

Blood pressure measurement: Systolic and diastolic blood pressure was measured non-invasively in conscious mice, using a tail-cuff system warmed to 37°C (VisitechBP-2000 Blood Pressure Analysis System). Mice are habituated to the device for 7-10 days and underwent two cycles of measurements per day for 3 days for blood pressure determination.

2-NBDG uptake assay: C2C12 myotubes with indicated gene transfer are incubated with serum-free DMEM for 12 h, and then maintained in Krebs-Ringer phosphate buffer (in mM: 128 NaCl, 1.4 CaCl₂, 1.4 MgSO4, 5.2 KCI, 10 Na₂HPO₄, and 2 sodium pyruvate, pH7.4) for 30 min at 37°C, and subsequently treated with 0.1 uM insulin and 100 uM 2-NBDG (Invitrogen) for 6 h at 37°C. Then, the cells are washed three times in ice-cold PBS, digested with 0.5% trypsin, centrifuged at 1,000 r.p.m. for 5 min, and then re-suspended in PBS. Finally, the FL1 fluorescence value was measured in a FACS Calibur Flow Cytometer (BD).

Metabolic measurements: For GTTs, mice are fasted overnight (for 16 hours) and then injected intraperitoneally (i.p.) with D-glucose (2 g kg⁻¹ body weight). For ITTs, mice are randomly fed and injected i.p. with bovine insulin (0.75Ukg⁻¹ body weight, Sigma-Aldrich). To assay glucose-stimulated insulin release, mice fasted for 16 h are injected i.p. with D-glucose (2 g kg⁻¹). Inventor collected blood from a tail vein before injection and at different time points after injection (as indicated in the figures). Glucose and insulin concentrations are measured with an AccuCheck blood glucose meter (Roche Diagnostics Inc.) and ELISA kits from Linco Research (catalogue number EZRMI-13K), respectively. Serum triglyceride and cholesterol concentrations are measured with kits from Wako Diagnostics (catalogue number 290-63701 and 294-65801, respectively).

For metabolic rate analysis, mice are housed individually under a 12-h light/dark cycle. A comprehensive animal metabolic monitoring system (CLAMS; Columbus Instruments) was used to evaluate oxygen consumption (VO2) and carbon dioxide production (VCO2) continuously over a 72-h period. Energy expenditure was calculated using the formula: energy expenditure= (3.815+1.232VO2/VCO2)×VO2.

Differences between groups are examined for statistical significance. Statistics: using one-way analysis of variance (ANOVA) or repeated-measures ANOVA, when appropriate, with the Bonferroni post-test. All P-values below 0.05 are considered significant. Data are expressed as mean±s.e.m.

The present invention also provides an animal-expressing vector, which are inserted with the gene sequence of MG53 mutant of claims, the vector may be an adenoviral vector or pcDNA4/TO/Myc-His B.

The present invention also provide an animal cell, which is transfected with animal-expressing vector as claimed, the animal cell may be C2C12 myotubes.

The present invention also provide a use of the pharmaceutical composition in treating myocardial injury disease including insulin resistance induced by myocardial injury, further in treating diseases including myocardial ischemia injury, myocardial ischemia/reperfusion injury, myocardial infarction, heart failure, cardiac arrhythmia and cardiac rupture. Particularly, the present invention provides a use of pharmaceutical composition comprising MG53 mutant in treating metabolic disorders including insulin resistance, obesity, diabetes. More preferentially, the present invention provides a use of pharmaceutical composition comprising MG53 mutant in regulating blood pressure.

A use of pharmaceutical composition comprising MG53 mutant means a use of pharmaceutical composition comprising MG53 mutant in treating myocardial injury, the MG53 mutant may be MG53C14A, or

A use of pharmaceutical composition comprisingMG53 mutant means a use of pharmaceutical composition comprising MG53 mutant in treating myocardial injury, the MG53 mutant may be MG53C29A, or

A use of pharmaceutical composition comprisingMG53 mutant means a use of pharmaceutical composition comprising MG53 mutant in treating myocardial injury, the MG53 mutant may be MG53C34A.

### Brief Description of the Drawings

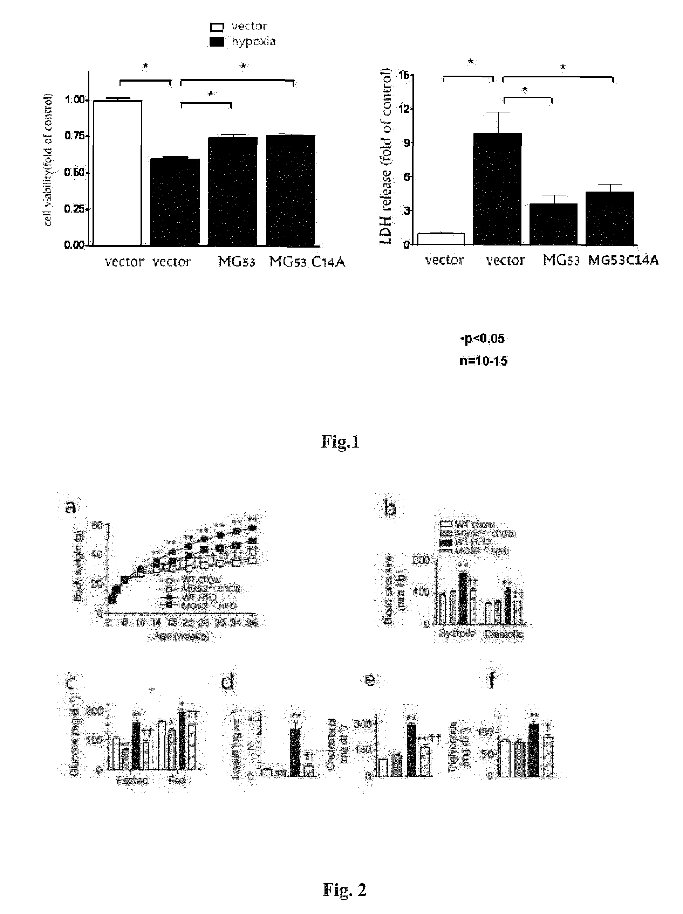

**Fig 1****: MG53, MG53C14A and MG53ΔRING protect the myotubes from the injury induced by hypoxia.**
   ATP assay (left)and extracellular LDH release(right), cultured myotubes subjected to hypoxia shows that MG53 or MG53C14A overexpression have more cell survivals as well as MG53 RING deletion does, while the vector control reveals significant cell death.
**Fig 2****: MG53 ablation protects mice against diet-induced metabolic syndrome.**
   a. Bodyweight of wild-type (WT) and MG53 ablation mice on chow or the HFD at indicated time points. b-e. blood pressure, glucose, insulin, cholesterol, triglyceride of wild-type (WT) and MG53 ablation mice on chow or the HFD for 35 weeks.

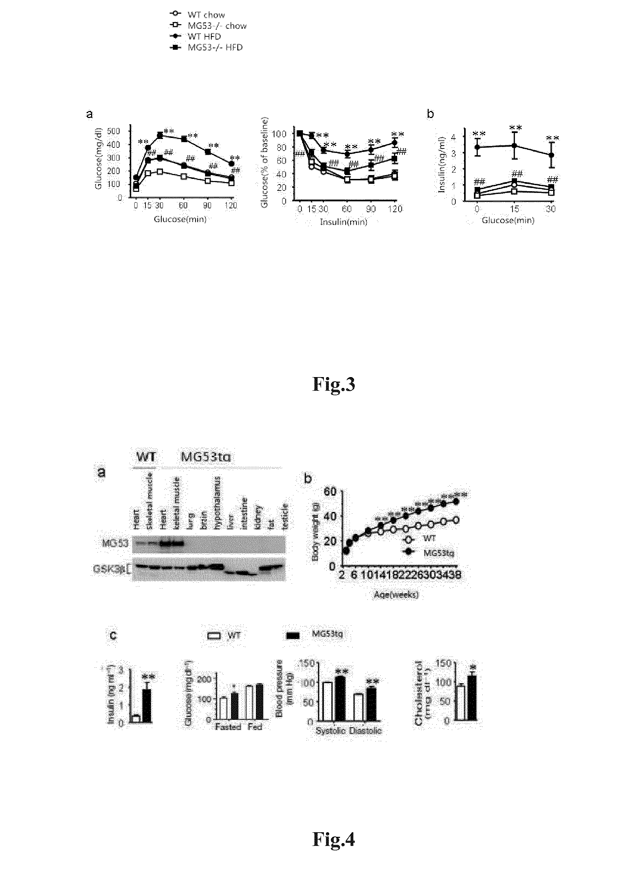

**Fig 3****: MG53 ablation blocks diet-induced systemic insulin resistance.**
   a. Glucose (left) and insulin (right) of wild-type (WT) and MG53 ablation mice on chow or the HFD for 30 weeks. b. Glucose stimulated changes in serum insulin concentration of wild-type (WT) and MG53 ablation mice on chow or the HFD for 30 weeks.
**Fig 4****: MG53^{tg} triggers obesity and metabolic syndrome**
   a. Representative western blots show that the MG53 expression in different tissues of MG53^{tg} mice. b. Body weight of wild-type (WT) and MG53^{tg} mice on chow at indicated time points. c insulin, blood glucose level, blood pressure and cholesterol of wild-type (WT) and MG53^{tg} mice on chow for 38 weeks.

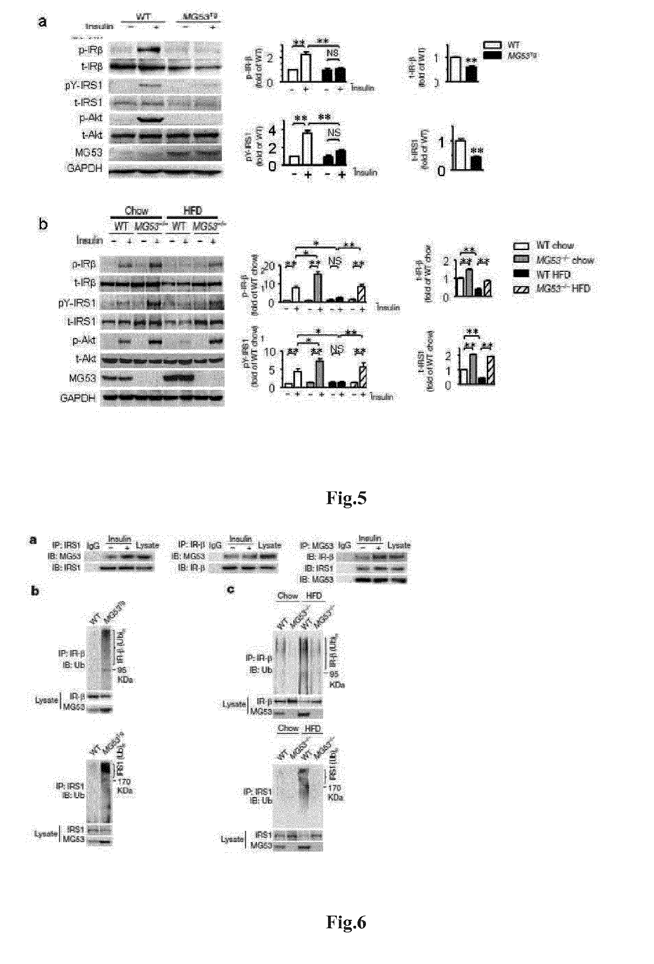

**Fig 5****: MG53 ablation blocks the HFD-induced insulin resistance**
   a. Representative western blots (left) and averaged data (right) showing insulin-induced tyrosine phosphorylation of insulin receptor-β subunit (IR-β) and IRS1, serine phosphorylation of the Akt and their total protein levels in skeletal muscle from wild-type andMG53^{Tg} mice at the age of 38 weeks. b. Representative western blots (left)and averaged data(right) showing insulin-induced tyrosine phosphorylation of insulin receptor-β subunit (IR-β) and IRS 1, serine phosphorylation of Akt and their total protein levels in skeletal muscle from MG53^{-/-}mice and on chow or the HFD for 35 weeks.
**Fig 6****: MG53 E3 ligase activates ubiquitination of the insulin receptor and IRS1.**
   a. Co-immunoprecipitation of MG53 with insulin receptor-β and IRS1 in skeletal muscle from wide-type mice.
   b. Ubiquitination of insulin receptor (upper) and IRS1 (lower) in skeletal muscle from wild-type andMG53^{Tg} mice at 38 weeks of age.
   c. Ubiquitination of insulin receptor (upper) and IRS1 (lower) in skeletal muscle from wild-type andMG53^{-/-} mice at 38 weeks of age.

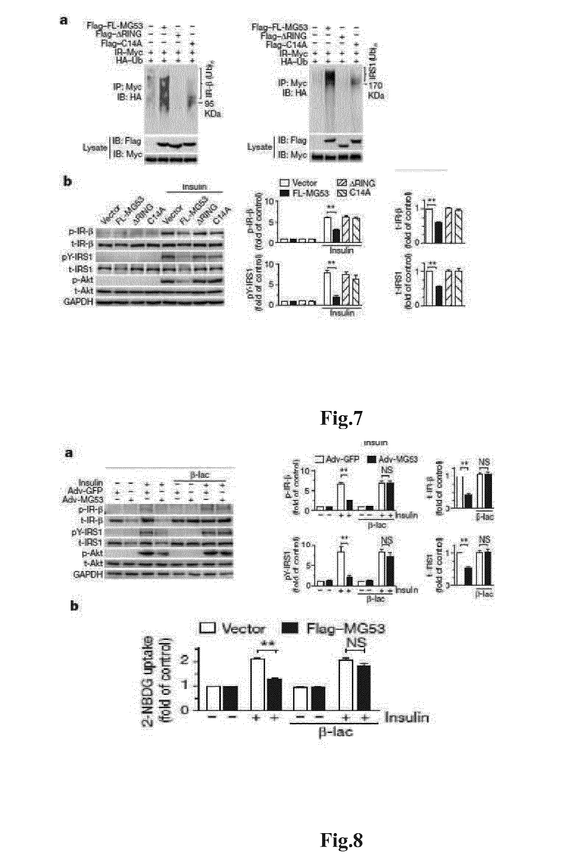

**Fig 7****: RING domain deletion MG53 and MG53C14A fail to activate the ubiquitination of IR and IRS1 and block the insulin signaling.**
   a. C2C12 myotubes expressing full-length MG53 (FL-MG53) activates ubiquitination of insulin receptor (left) and IRS1 (right), which is not seen in RING domain deletion (Flag-RNG) MG53 and C14A mutant (Flag-C14A).
   b. Representative western blots(left) and averaged data(right) shows that C2C12 myotubes expressing full-length MG53 may significantly block insulin-induced phosphorylation of IR, IRS1 and Akt, reduce total IR and IRS1; which is not seen in RING domain deletion MG53 and MG53C14A.
**Fig 8** **Protease inhibitor suppresses the MG53-mediated insulin signaling pathways.**
   a. Representative western blots (left) and averaged data (right) showing that Adv-MG53 overexpression blocked the MG53-mediated suppression of phosphorylation of insulin receptor and IRS1 and Akt, and downregulated the insulin receptor-β and IRS1 in C2C12 myotubes.
   b. Insulin stimulation upregulates the uptake of 2-NBDG in C2C12. Overexpression of Flag-MG53 suppresses the insulin-mediated 2-NBDG uptake in C2C12. While β-Lac abrogated MG53-mediated suppression of 2-NBDG uptake in C2C12 myotubes.

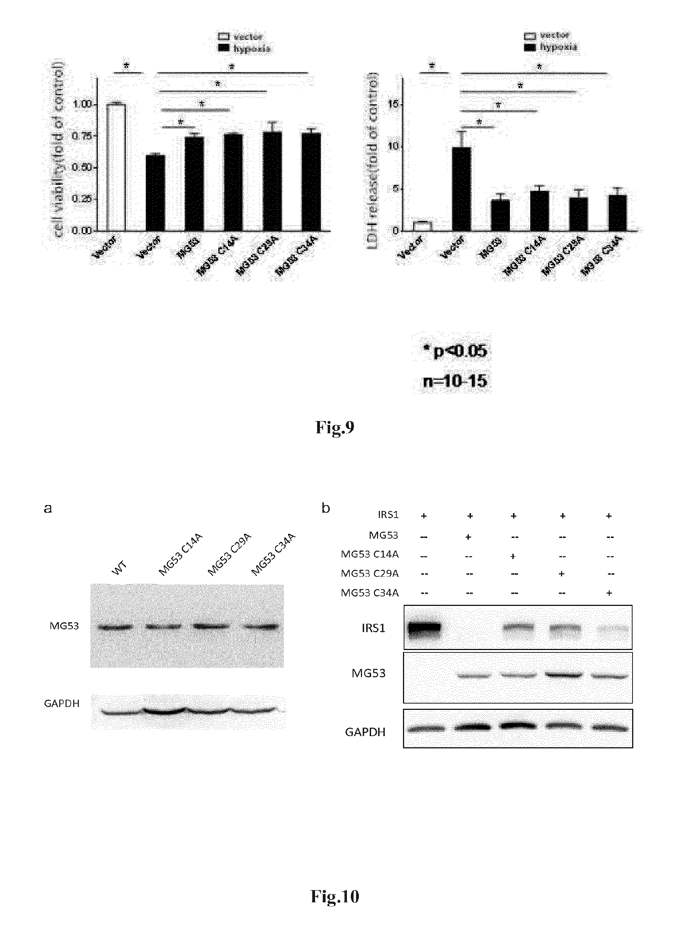

**Fig 9****: MG53C29A and MG53C34A protect the myotubes from injury induced by hypoxia.**
   ATP assay (left)and extracellular LDH release(right), cultured myotubes subjected to hypoxia shows that the MG29A or MG53C34A overexpression have more cell survivals as well as MG53 or MG53C14A, while the vector control reveals significant cell death.
**Fig10****. MG53C29A and MG53C34A trigger no IRS1 degradation.**
   a. Cultured C2C12 cells transfected with wild-type MG53 or MG53C14A, MG5329A, MG53C34A share the similar level of expressions respectively as that of MG53 or MG53 mutant.
   b. In cultured C2C12, MG53 overexpression reduce the IRS1 as MG53C14A does, but MG53C29A and MG53C34A triggers no IRS1 degradation.

### Embodiments

The examples hereinafter adopt an Easy Mutagenesis System kit (TransGen Biotech, China) for a point-mutation.

### Example1. The mutation of the MG53C14A (MG53 mutant protein, or MG53 mutant: MG53C14A).

1. Design the upperstream and downstream primers, which contains the mutation site, and the length of overlapping region is 18-27bp; Primers:
   C14A primer 1:
      5'-gaactgtccgccccactgtgcttgcagctg-3'
   C 14A primer 2:
      5'- agcacagtggggcggacagttcctgacgca-3'
2. Amplify the MG53 gene sequence by PCR reaction with DNA polymerase with 200ng wild-type MG53 plasmid or cDNA of MG53 as the template. The PCR product is confirmed by agrose gel electrophoresis. The to-be-mutated plasmid is wild-type MG53 plasmid.
   PCR program: 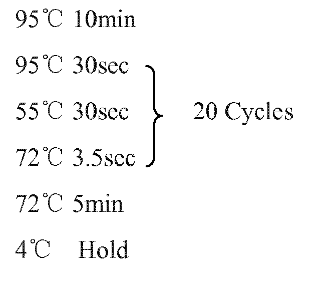
3. The PCR product is digested by restrictive enzyme DpnI, where the reaction is as follows: add 1ul DpnI to 10ul PCR product at 37 °C for overnight digestion.
4. Transform the restrictive cut product into E.coli competent TOP10: thaw the competent TOP10, followed by adding PCR product into the cell and incubate on ice for 30min, then heatshock the mixture at 42°C for 60s, followed by adding LB medium and shaking culture for 45min, then spray the cells on LB medium plate with antibiotics and overnight incubate at 37°C.
5. Select the single colony for DNA sequencing, the positive colony means successful construction of the MG53 mutant plasmid.

*Reference documents: Strata Gene 'QuikChange Site-directed Mutagenesis kit. TransGen Biotech Easy Mutagenesis System. Detailed Protocol:*
*http:*//*www.transgen.com.cn*/*uploadfile*/*201111*/*20111109161357731.pdf.*

### Example 2. The mutation of the MG53C17A (MG53 mutant protein, or MG53 mutant: MG53C14A).

The method of constructing the MG53C17A:
Primers:
   C 17A primer 1:
      5'- gcccactggccttgcagctgttcgatgcgc-3'
   C17A primer 2:
      5'- cagctgcaaggccagtgggcaggacagttc-3'
   Other steps are similar to that of C14A.

### Example 3. AMG53 mutant: MG53C29A.

The method of constructing the MG53C29A:
Primers:
   C29A primer 1:
      5'- acggctgaggctggccacagtttctgccgt-3'
   C29A primer 2:
      5'- actgtggccagcctcagccgtcactggcgc-3'
   Other steps are similar to that of C14A, or refer to example 1 or 2.

### Example 4. AMG53mutant: MG53C34A.

The method of constructing the MG53C34A:
Primers:
   C34A primer 1:
      5'- cacagtttcgcccgtgcctgcctgatccgg-3'
   C34A primer 2:
      5'- gcaggcacgggcgaaactgtggccacactc-3'
   Other steps are similar to that of C14A.

### Example 5. A MG53mutant: MG53C37A.

The method of constructing the MG53C37A:
Primers:
   C37A primer 1:
      5'-tgccgtgccgccctgatccgggtggcaggg-3'
   C37A primer 2:
      5'- ccggatcagggcggcacggcagaaactgtg-3'
   Other steps are similar to that of C14A. Or refer to the examples 1~4. Sequence alignment is shown in Fig 9.

### Example 6. A MG53mutant : MG53C53A.

The method of constructing the MG53C53A:
Primers:
   C53A primer 1:
      5'- acagttgccgctccctgttgtcaggcacct-3'
   C53A primer 2:
      5'- acaacagggagcggcaactgtgccgtccgc-3'
   Other steps are similar to that of C14A.

### Example 7. A MG53mutant: MG53C56A.

The method of constructing the MG53C56A:
Primers:
   C56A primer 1:
      5'-tgtccctgtgctcaggcacctacacggccg-3'
   C56A primer 2:
      5'- aggtgcctgagcacagggacaggcaactgt-3'
   Other steps are similar to that of C14A.

### Example 8. A MG53 mutant : MG53C14G.

The method of constructing the MG53C14G:
Primers:
   C14G primer 1:
      5'-gaactgtccggcccactgtgcttgcagctg-3'
   C14G primer 2:
      5'- agcacagtgggccggacagttcctgacgca-3'
   Other steps are similar to that of C14A.

### Example 9. A MG53 mutant: MG53C17G.

The method of constructing the MG53C17G:
Primers:
   C 17G primer 1:
      5'- gcccactgggcttgcagctgttcgatgcgc-3'
   C17G primer 2:
      5'- cagctgcaagcccagtgggcaggacagttc-3'
   Other steps are similar to that of C14A.

### Example 10. A MG53 mutant: MG53C29G.

The method of constructing the MG53C29G:
Primers:
   C29G primer 1:
      5'- acggctgagggtggccacagtttctgccgt-3'
   C29G primer 2:
      5'- actgtggccaccctcagccgtcactggcgc-3'
Other steps are similar to that of C14A.

### Example 11. A MG53mutant: MG53C34G.

The method of constructing the MG53C34G:
Primers:
   C34G primer 1:
      5'- cacagtttcggccgtgcctgcctgatccgg-3'
   C34G primer 2:
      5'- gcaggcacggccgaaactgtggccacactc-3'
   Other steps are similar to that of C14A.

### Example 12. A MG53mutant : MG53C37G.

The method of constructing the MG53C37G:
Primers:
   C37G primer 1:
      5'-tgccgtgccggcctgatccgggtggcaggg-3'
   C37G primer 2:
      5'- ccggatcaggccggcacggcagaaactgtg-3'
   Other steps are similar to that of C14A.

### Example 13. A MG53mutant: MG53C53G.

The method of constructing the MG53C53G:
Primers:
   C53G primer 1:
      5'- acagttgccggtccctgttgtcaggcacct-3'
   C53G primer 2:
      5'- acaacagggaccggcaactgtgccgtccgc-3'
   Other steps are similar to that of C14A.

### Example 14. A MG53mutant: MG53C56G.

The method of constructing the MG53C56G:
Primers:
   C56G primer 1:
      5'- tgtccctgtggtcaggcacctacacggccg-3'
   C56G primer 2:
      5'- aggtgcctgaccacagggacaggcaactgt-3'
   Other steps are similar to that of C14A.

### Example 15. A MG53mutant: MG53C14L.

The method of constructing the MG53C14L:
Primers:
   C14L primer 1:
      5'-gaactgtccctcccactgtgcttgcagctg-3'
   C14L primer 2:
      5'- agcacagtgggagggacagttcctgacgca-3'
   Other steps are similar to that of C14A.

### Example 16. A MG53mutant: MG53C14V.

The method of constructing the MG53C14V:
Primers:
   C 14V primer 1:
      5'-gaactgtccgtcccactgtgcttgcagctg-3'
   C 14V primer 2:
      5'- agcacagtgggacggacagttcctgacgca-3'
   Other steps are similar to that of C14A.

### Example 17. A MG53mutant: MG53C14I.

The method of constructing the MG53C14I:
Primers:
   C14I primer 1:
      5'-gaactgtccatcccactgtgcttgcagctg-3'
   C14I primer 2:
      5'- agcacagtgggatggacagttcctgacgca-3'
   Other steps are similar to that of C14A.

### Example 18. A MG53mutant: MG53C14P.

The method of constructing the MG53C14P:
Primers:
   C14P primer 1:
      5'-gaactgtccccaccactgtgcttgcagctg-3'
   C14P primer 2:
      5'- agcacagtggtggggacagttcctgacgca-3'
   Other steps are similar to that of C14A.

### Example 19. A MG53mutant: MG53C17L.

The method of constructing the MG53C17L:
Primers:
   C17L primer 1:
      5'- gcccactgctcttgcagctgttcgatgcgc-3'
   C17L primer 2:
      5'- cagctgcaagagcagtgggcaggacagttc-3'
   Other steps are similar to that of C14A.

### Example 20. A MG53mutant: MG53C29L.

The method of constructing the MG53C29L:
Primers:
   C29L primer 1:
      5'-acggctgagcttggccacagtttctgccgt-3'
   C29L primer 2:
      5'-actgtggccaagctcagccgtcactggcgc-3'
   Other steps are similar to that of C14A.

### Example 21

MG53 mutant expression by transfecting MG53 mutant plasmid with ScreenFect®A into HEK293T cells.

A great amount of plasmid (Flag-MG53 or Flag-C14A MG53) are transfected by ScreenFectA (Incella) into HEK293T cultured on 60mm dish with 90% confluence, the protocol is as follows:
(1) Remove the cell medium and replace with 2ml DMEM.
(2) Mix 250ul dilution buffer with 5mg plasmid in the eppendorff tube A
(3) Mix 250ul dilution buffer and 50ul ScreenFectA in the eppendorff tube B, vortexing.
(4) 5 min later, mix the tube A and tube B together, and store it at room temperature for 30min.
(5) Transfer the mixture into the cells for 6h, the medium is changed to the general medium.

48 hours later, the cells are lysed with cell lysing buffer and the MG53 mutant proteins are separated by SDS-PAGE, finally the protein level is determined by Flag antibody after membrane transferring.

### Example 22. MG 53 RING deletion by PCR method:

PCR primers:
dR-F: 5'-ataggtaccgccaccatggcacctacacggccgcagg-3'
dR-R: 5'-atactcgaggcggcctgttcctgctccggc-3'

Restriction sites are for the KpnI and XhoI with the whole sequence of MG53 plasmid as the template , the reaction system is as follows:

| | |
|---|---|
| Template DNA: | 1 uL (100ng/uL) |
| Primer dR-F: | 1 uL |
| Primer dR-R: | 1 uL |
| KAPA Hot start PCR mix: | 50 uL |
| H₂O: | 47 uL |

The PCR program is as follows:

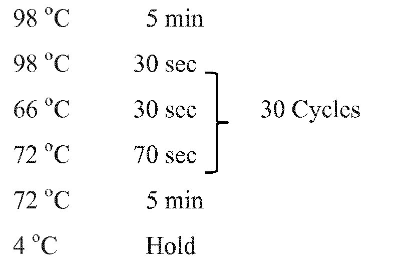

The PCR product and pcDNA4/TO/myc-His B are digested by restrictive enzyme KpnI/XhoI, retrieved by agrose gel electrophoresis, followed by ligation with T4 Ligase. The constructs are transformed into E.coli TOP10 for cell culture. Select the single colony for DNA sequencing, the positive colony means successful construction of the MG53 RING deletion plasmid.

The protein sequence of dRING construct: from the 58^{th} Ala in the N-terminus to the end of the MG53 with the Myc-tagged C-terminus.

### Example 23 The cardioprotection capacities of MG53 mutants and wild type MG53.

MG53C14A, MG53C29A, MG53C34A plasmids are constructed by means of example 1, 3, 4, then are transfected into myotubes under hypoxia according to example 21. ATP assay (left)and extracellular LDH release(right), cultured myotubes induced by hypoxia shows that the MG53C29A or MG53C34A overexpression both have more cell survival as well as MG53 or MG53C14A do, while the vector control reveals significant cell death. See Fig 9.

Cell Hypoxia: cells are cultured in RPMI1640/5 % FBS for 48 hours. Then, the medium was replaced with serum-free RPMI1640 saturated with 95% N₂ /5% CO₂, and cells are placed in a 37 °C airtight box saturated with 95% N₂/5% CO₂ for various periods of time. O₂ concentrations are < 0.1 % (Ohmeda oxygen monitor, type 5120). For normoxia controls, culture medium was changed to RPMI1640 / 5% FCS, and cells are placed in a 37°C / 5% CO₂ incubator before analysis.

### Determination of Myocardial Injury by:

CellTiter-GloLuminoescent Cell Viability Assay (Cat#G7571, Promega) was used for ATP assay. Details of the method: 1. Mix CellTiter-Glo Substrate (1 tube) and CellTiter-Glo Buffer (1 tube), thawed to room temperature prior to use; 2. Take the cell sample out of the incubator and equilibrate to room temperature prior to use. 3. Adding equal amount of ATP reagent to each wells of cell plate filled with the cell sample; 4. Gentle shake the cell plates for 2 minutes (the sample in this step contains cells, medium and ATP reagents); 5. Equilibrate at room temperature for 10min; 6. transfer the sample into the plate for Luminescent Assay.

LDH was spectrophotometrically assayed with the use of a kit (LDH0360, Shanghai ,China), the protocol is as follows:
1. Take out of reagents from the box, mix the reagent 2 with the reagent 1 with a ratio of 1:5 , store at the temperature between 2 and 8°C prior to use;
2. Add each well with 40ul sample;
3. Add each well with 200ul reaction mixture, measure the absorbance of the samples at 340nm.

### Example 24 The expression of the MG53 mutants and wild type MG53 in C2C12 myotubes.

MG53C14A, MG53C29A, MG53C34A plasmids are constructed by means of example 1, 3, and 4. C2C12 myoblasts (from Cell Resource Center, IBMS, CAMS/PUMC) are cultured at 37°C under 5% CO₂ in Dulbecco's modified Eagle's medium (DMEM) supplemented with 10% fetal bovine serum (Sigma-Aldrich), 0.11 g/L sodium pyruvate, and 1% penicillin-streptomycin. When C2C12 myoblasts reached 90% confluence, inventor performed gene transfer by adenoviral infection or plasmid transfection. After gene transfer, cells are cultured in DMEM (2% horse serum) for 4 days to differentiate into myotubes.

The expression level of wild type MG53, MG53C14A, MG5329A and MG53C34A in C2C12 are similar to that of MG53 or MG53 mutants. Please see Fig 10a for explanation.

### Example 25 IRS1 Degradation by wild type MG53 and the MG53 mutants.

MG53C14A, MG53C29A, MG53C34A plasmids are constructed by means of example 1, 3, and 4. C2C12 myoblasts (from Cell Resource Center, IBMS, CAMS/PUMC) are cultured at 37°C under 5% CO₂ in Dulbecco's modified Eagle's medium (DMEM) supplemented with 10% fetal bovine serum (Sigma-Aldrich), 0.11 g/L sodium pyruvate, and 1% penicillin-streptomycin. When C2C12 myoblasts reached 90% confluence, the inventor performed gene transfer by adenoviral infection or plasmid transfection. After gene transfer, cells are cultured in DMEM (2% horse serum) for 4 days to differentiate into myotubes.

Co-immunoprecipitation: Tissues or cells are lysed in lysis buffer A (30mM HEPES at pH7.6 100mM NaCl, 0.5% Nonidet P-40, and protease inhibitors mixture) for 30 min at temperature 4°C, and the lysates are centrifuged at 13,000 r.p.m. for 10 min at 4°C. remove the precipitates , the supernatant of total proteins is ready for use. Wash the protein A beads by ice-cold 1×PBS, then the beads are centrifuged at 4,000 r.p.m for 2min ,removed the supernatant, repeat the wash for 3 times , remove the PBS, mix the beads with the protein supernatant and 0.5ug anti-IRS1, the mixture are incubated for 4h at 4°C. Then repeat the step of wash as described previously for 3 times, the beads mix with 1Xloading buffer, and are boiled for 5min at 100°C, then centrifuged at 13,000 r.p.m. for 10 min, the supernatant are loaded on SDS-PAGE and transfer to PVDF. Analysis of tyrosine phosphorylation of IRS1 was performed by immunoprecipitation of IRS 1 with anti-IRS 1 from total lysate, followed by western blot with anti-pTyr antibody (PY100). Other proteins are analyzed with specific antibodies.

In C2C12 cells, MG53 overexpression reduces the IRS level. Similar to MG53C14A, however, MG53C29A and MG53C34A cannot downregulate the IRS1, which suggests that MG53 mutants cannot provoke the insulin resistance and impose less impact on metabolic pathways in contrast to that of the MG53 overexpression. Please see Fig 10b for further explanation.

It is confirmed by experiments that the MG53 mutants, including MG53C14A, MG53C17A, MG53C29A, MG53C34A, MG53C37A, MG53C53A, and MG53C56A, all perform the effect of cardiac protection. MG53 overexpression will protect the hearts, but accompany the side effects such as insulin resistance, obesity diabetes and other metabolic syndromes. The 14^{th}, 17^{th}, 29^{th}, 34^{th}, 37^{th}, 53^{th}, 56^{th} cysteines, especially the 14^{th} cysteine, are essential for MG53 in leading to the side effects above. That means, the mutation of MG53 in the cysteine sites as described above, especially 14^{th} cysteine, will protect the hearts and avoid the side effects as described above. To be exact, the mutants in MG53C14A will protect the hearts and avoid the side effects as described above.

The sequence of the mutant as described in this application, including the MG53 mutants used in the embodiments, could be selected from primates (for instance, human), rats and mice. The wild-type MG53 (TRIM72) sequence is selected from mice, of which the NCBI number is NM_001079932.3, other alternative may come from as follows:
Human sapiens: mRNA :NM_001008274.3, and CDS: NP_001008275.2;
Rats: NM_001077675.1 and CDS: NP_001071143.1;
Monkeys: mRNA: XM_001112866.2 and CDS: XP_001112866.1.

The gene source shall be not limited to the scope of the species as mentioned above, where all MG53 expressing species shall be taken into account and the correspondent MG53 mutants are based on the sequences described above.

The term 'MG 53 mutant' referred in this application means the mutated MG53 protein or MG53 mutant protein.

What is described above is aimed to further illustrate the invention without confined to the scope of it. The disclosed embodiments may be improved or changed within the scope and spirit of the present invention by the person skilled in the relevant field.

## Claims

1. A MG53 mutant, wherein one or two or more than two of the seven cysteine sites of the N-terminal RING domain are substituted by non-polar amino acids; the seven cysteines situates in the 14^{th}, 17^{th}, 29^{th}, 34^{th}, 37^{th}, 53^{th}, 56^{th} sites of the RING domain.

2. A MG53 mutant of claim 1, wherein the amino acids may be alanine, glycine, valine, leucine, isoleucine, proline, phenylalanine, tryptophan or methionine.

3. A MG53 mutant of claim 2, wherein the amino acids may be alanine, glycine, leucine, proline, valine or isoleucine.

4. A MG53 mutant gene, wherein the gene sequence of MG53 mutant encodes the protein of MG53 mutant of claim 1.

5. A MG53 mutant of claim 3, wherein the non-polar amino acid may be alanine, the MG53 mutant may be any of MG53C14A, MG53C17A, MG53C29A, MG53C34A, MG53C37A, MG53C53A or MG53C56A.

6. A method of constructing the mutation of MG53, wherein the method adopts a point-mutation kit to mutate the sequence of wild-type MG53 plasmid, to obtain the MG53 mutant plasmids.

7. A method of the mutation of claim 6, wherein the method adopts a point-mutation kit to mutate the whole sequence of wild-type MG53 plasmid, to obtain the mutated MG53 plasmid with 14th cysteine to non-polar amino acid mutation.

8. A method of mutation of claim 6, wherein the mutation protocol is:
(1) Design the upperstream and downstream primers, which contains the mutation site, and the length of overlapping region is 18-27bp;
(2) Amplify the MG53 gene sequence by PCR reaction with DNA polymerase with wild-type MG53 plasmid or cDNA of MG53 as the template. The PCR product is confirmed by agrose gel electrophoresis. The to-be-mutated plasmid is wild-type MG53 plasmid.
(3) The PCR product is digested by restrictive enzyme DpnI.
(4) Transform the restrictive cut product into E.coli competent TOP10: thaw the competent TOP10, followed by adding PCR product into the cell and incubate on ice, followed by adding LB medium and shaking culture, then spray the cells on LB medium plate with antibiotics, select the single colony for DNA sequencing, the positive colony means successful construction of the MG53 mutant plasmid.
(5) The MG53 mutant protein will be obtained by transfecting the mutation MG53 plasmid into cells by ScreenFectA or Lipofectamine.

9. An animal expressing vector, wherein the vector is inserted with the MG53 mutant gene of claim 8.

10. An animal expressing vector of claim 9, wherein the vector may be adenoviral vector.

11. An animal expressing vector of claim 10, wherein the vector may be pcDNA4/TO/Myc-His B.

12. An animal cell, wherein the cell is transfected with the animal expressing vector of claim 9 or 10.

13. An animal cell of claim 12, wherein the cell may be C2C12 myotube cell.

14. A use of pharmaceutical composition comprising the MG53 mutants in claim 1 in treating myocardial injury.

15. An use of claim 14, wherein the pharmaceutical composition in treating myocardial injury disease including insulin resistance induced by myocardial injury, including myocardial ischemia injury, myocardial ischemia/reperfusion injury, myocardial infarction, heart failure, cardiac arrhythmia and cardiac rupture.

16. A use of claim 14, wherein pharmaceutical composition comprising MG53 mutant in treating metabolic disorders, including insulin resistance, obesity and diabetes.

17. A use of claim 14, wherein pharmaceutical composition comprising MG53 mutant in regulating blood pressure.

18. A use of any of the claims 14-17, wherein the MG53 mutant may be MG53C 14A, and the use of pharmaceutical composition comprising MG53C14A in treating myocardial injury.

19. A use of any of the claims 14-17, wherein the MG53 mutant may be MG53C29A, and the use of pharmaceutical composition comprising MG53C29A in treating myocardial injury.

20. A use of any of the claims 14-17, wherein the MG53 mutant may be MG53C34A, and the use of pharmaceutical composition comprising MG53C34A in treating myocardial injury.
